# DEMANDE DE BREVET EUROPEEN

(11) **EP 4 652 956 A1**
(43) Date de publication de la demande: **26.11.2025**
(21) Numéro de dépôt: 24208393.9
(22) Date de dépôt: 23.10.2024
(51) Int. Cl.: A61B 50/30, A61B 50/33, A61C 19/02, A61B 17/00, A61G 15/16

(54) **PLATEAU D'EXAMEN COMPOSTABLE POUR LES DENTISTES**

(30) Priorité: 20.05.2024 FR 2405135
(71) Demandeur: Compack, 75017 Paris (FR)
(72) Inventeur: GAILLIARDOT, Nathalie, 27730 BUEIL (FR); BAROUZDIN, Alexandre, 75003 PARIS (FR)
(74) Mandataire: Argyma

(57) **Abrégé**

La présente invention vise un plateau (1) d'examen jetable pour le domaine médical, en particulier pour le domaine dentaire, comprenant deux faces principales (10, 11) opposées, à savoir une face supérieure (10) et une face inférieure (11), ledit plateau étant monobloc et comportant un relief, formé dans l'une des faces principales (10 ; 11) et délimitant des compartiments de réception (12, 120, 121, 122), lesdits compartiments de réception (12, 120, 121, 122) étant configurés pour recevoir des instruments médicaux ; ledit plateau (1) étant formé de fibre de sucre de canne et de fibre de bambou et étant configuré pour être compostable.

## Description

### DOMAINE TECHNIQUE

La présente invention concerne un plateau pour le domaine du matériel médical, en particulier pour le domaine dentaire.

L'invention a trait à un plateau d'examen compostable pour les dentistes.

### ETAT DE LA TECHNIQUE

Il est connu de l'état de la technique un plateau d'examen pour dentiste, jetable et en matière plastique. Un tel plateau permet au dentiste d'entreposer ses instruments et/ou des produits qu'il souhaite utiliser lors d'une intervention sur un patient par exemple.

Un tel plateau comprend soit un compartiment unique, s'étendant sur l'ensemble d'une des surfaces du plateau, soit une pluralité de compartiments. La pluralité de compartiment permet de délimiter des zones sur le plateau et donc de séparer les instruments et/ou produits entre eux.

Toutefois, un tel plateau d'examen pour dentiste est à usage unique. Après chaque utilisation, il est jeté. Le matériau plastique qui le compose n'étant pas recyclable, et à usage unique pour des raisons de propreté et stérilité, l'utilisation d'un tel plateau est particulièrement polluant, tant par sa composition que par le nombre de plateau jeté sur une durée déterminée.

Ainsi, l'invention vise à résoudre les problèmes susmentionnés en proposant un plateau d'examen compostable pour dentiste, répondant aux exigences médicales tel que le besoin de stérilité, commode, économique et pratique d'utilisation pour les dentistes.

### PRESENTATION DE L'INVENTION

Plus précisément, l'invention a pour objet un plateau d'examen pour le domaine médical, en particulier pour le domaine dentaire, comprenant deux faces principales opposées, à savoir une face supérieure et une face inférieure, ledit plateau étant monobloc et comportant un relief, formé dans l'une des faces principales et délimitant des compartiments de réception, lesdits compartiments de réception étant configurés pour recevoir des instruments médicaux ; ledit plateau étant formé de fibre de sucre de canne et de fibre de bambou et étant configuré pour être compostable.

Le plateau est formé d'un mélange de résidus fibreux, la bagasse et la fibre de bambou. La bagasse est issue du broyage de la canne à sucre et présente l'avantage d'être biodégradable, tout comme la fibre de bambou. En particulier, la bagasse et la fibre de bambou se décomposent naturellement bien plus rapidement que le plastique utilisé dans les plateaux connus. Le plateau selon l'invention a, ainsi, un impact environnemental réduit. De plus, les compartiments de réception formés dans le plateau permettent de délimiter des zones sur le plateau et donc de séparer les instruments médicaux et/ou produits entre eux utilisés par le dentiste ou professionnel médical. Le plateau permet donc une utilisation aisée, commode, tout en ayant un impact environnemental réduit.

Les plateaux selon l'invention sont destinés à être jetés dans des collecteurs de DASRI (pour déchets d'activités de soins à risques infectieux). Ils sont ensuite prévus pour être incinérés et pour émettre à ce titre moins de CO2, notamment 63% de moins, qu'un plateau en plastique comme ceux utilisés à ce jour par les praticiens.

Avantageusement, le plateau est formé de 70% de fibre de sucre de canne et de 30% de fibre de bambou.

De telles proportions permettent d'augmenter considérablement la solidité dudit plateau.

Avantageusement, des compartiments de réception juxtaposés sont séparés par une paroi présentant une denture alternant dent et creux sur toute la longueur de la paroi.

Avantageusement encore, chaque paroi saille de la face principale formant le fond des compartiments de réception, ladite paroi étant creuse, un logement étant formé dans chaque paroi débouchant sur l'autre face principale.

Avantageusement, au moins trois compartiments de réception sont juxtaposés et séparés par deux parois présentant chacune une denture, la denture de la première paroi étant en miroir de la denture de la seconde paroi, un premier creux de la première paroi en miroir d'un premier creux de la seconde paroi formant alors des points d'appui configuré pour recevoir un instrument médical.

L'instrument médical peut donc être disposé sur les points d'appui dans des creux en miroir de la première et de la seconde paroi. Ledit instrument est donc interposé entre deux dents sur ses deux points d'appui, ce qui lui permet d'être bloqué en position.

Avantageusement, des motifs sont formés dans la face principale formant le fond d'au moins une partie des compartiments, les motifs étant configurés pour créer des proéminences sur le fond des compartiments de réception.

De tels motifs permettent au plateau de présenter une fonction antidérapante, empêchant le glissement du matériel médical logé dans les compartiments de réception.

Avantageusement, dans lequel les compartiments de réception présentent des profondeurs différentes et des formes différentes et/ou comprennent un compartiment de réception ayant un fond lisse et/ou un compartiment de réception comprenant une pluralité d'alcôves, notamment deux paires d'alcôves de diamètre différent, et/ou un compartiment de réception formant un bloc à spatuler.

Les profondeurs et les formes différentes permettent de s'adapter à une pluralité d'instruments médicaux et donc de s'adapter au mieux aux besoins de l'utilisateur. Le compartiment de réception à fond lisse permet notamment de recevoir de l'alcool ou de la chlorhexidine. Le compartiment de réception disposant d'alcôves permet de recevoir du solvant, du vernis ou de la colle. Le bloc à spatuler permet au praticien de spatuler de la pâte ou de préparer de l'eugénate par exemple.

Avantageusement, le plateau comporte une portion de préhension plane, configurée pour permettre la saisie dudit plateau par un utilisateur, ladite portion de préhension étant formée par une portion plane des deux faces principales.

L'invention concerne également un procédé de fabrication d'un plateau précédemment décrit, comportant les étapes suivantes :
- modeler un mélange de 70% de pulpe de sucre de canne et de 30% de pulpe de bambou sur un moule métallique ;
- déshydrater ledit mélange par compression et exposition à une source de chaleur ;
- sécher ledit mélange au moyen de vapeur de sorte à obtenir un ensemble modelé à la forme requise ;
- exposer ledit ensemble aux rayons ultra-violets ; et
- découper ledit ensemble traité aux rayons ultra-violets aux dimensions requises de sorte à former ledit plateau (1).

L'invention concerne encore un ensemble de plateaux, comportant au moins deux plateaux identiques tels que précédemment décrits, dans lequel lesdits plateaux sont empilables les uns sur les autres, chaque paroi d'un premier plateau étant configurée pour être logée dans un logement d'une paroi d'un deuxième plateau et chaque compartiment de réception dudit deuxième plateau étant configuré pour être logé dans chaque compartiment de réception dudit premier plateau.

Les plateaux étant empilables les uns sur les autres, ils présentent un encombrement moindre pour leur transport et leur stockage.

### PRESENTATION DES FIGURES

L'invention sera mieux comprise à la lecture de la description qui va suivre, donnée uniquement à titre d'exemple, et se référant aux dessins annexés donnés à titre d'exemples non limitatifs, dans lesquels des références identiques sont données à des objets semblables et sur lesquels :
La figure 1 est une représentation schématique en perspective d'un plateau d'examen compostable selon l'invention, présentant une pluralité de compartiments de réception ;
La figure 2 est une de côté du plateau de la figure 1, présentant trois compartiments de réception identiques juxtaposés ;
La figure 3 est une vue de dessus du plateau de la figure 1, rendant visible l'ensemble des compartiments de réception du plateau ; et
La figure 4 est une autre vue de côté du plateau de la figure 1, présentant une pluralité de compartiments de réception différents.

Il faut noter que les figures exposent l'invention de manière détaillée pour permettre de mettre en oeuvre l'invention ; bien que non limitatives, lesdites figures servent notamment à mieux définir l'invention le cas échéant.

### DESCRIPTION DETAILLEE DE L'INVENTION

L'invention concerne un plateau d'examen 1 pour le domaine médical. En particulier, le plateau 1 est pour le domaine dentaire.

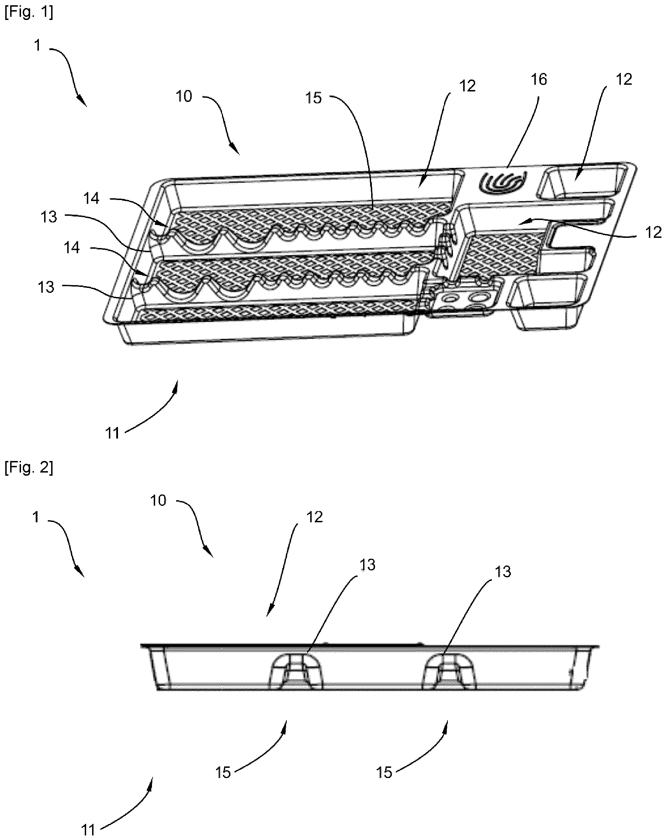

Le plateau 1, représenté sur la figure1, est un plateau 1 jetable pour dentiste, compostable et biodégradable.

Le plateau 1 présente deux faces principales 10, 11 opposées l'une à l'autre. En particulier, le plateau 1 présente une face supérieure 10 et une face inférieure 11, la face supérieure 10 et la face inférieure 11 étant opposées l'une à l'autre. La face inférieure 11 est destinée, en utilisation, à être posée sur un meuble par exemple, alors que la face supérieure 10 est destinée à être en contact avec du matériel médical.

Le plateau 1 est monobloc et comporte un relief formé dans l'une des faces principales 10, 11, ici la face supérieure 10. Le relief délimite au moins un compartiment de réception 12, 120, 121, 122. Selon le mode de réalisation illustré sur les figures, le plateau 1 comporte une pluralité de compartiments de réception 12, 120, 121, 122. Chaque compartiment de réception 12 est formé dans l'une des faces principales 10, 11 et saille de l'autre face principale 11, 10. En référence à la figure 1, chaque compartiment de réception 12, 120, 121, 122 est formé dans la face supérieure 10 et saille de la face inférieure 11.

Chaque compartiment de réception 12 est configuré pour recevoir des instruments médicaux, en particulier des instruments de dentiste ou d'orthodontiste par exemples. Il est évident que les compartiments de réception peuvent le cas échéant recevoir tout type de matériel médical, même autre que matériel dentaire.

Le plateau 1 est formé d'un mélange de résidus fibreux, la fibre de sucre de canne et la fibre de bambou. La fibre de sucre de canne, autrement appelée la bagasse est issue du broyage de la canne à sucre et présente l'avantage d'être compostable et biodégradable, tout comme la fibre de bambou. En particulier, la bagasse et la fibre de bambou se décomposent naturellement bien plus rapidement que le plastique utilisé dans les plateaux connus. Le plateau 1 selon l'invention a ainsi un impact environnemental réduit.

Avantageusement, le plateau 1 présente une épaisseur comprise entre 0.20 mm et 0.60 mm. Une telle épaisseur permet d'assurer la rigidité du plateau 1.

Selon un mode de réalisation de l'invention, le plateau 1 est formé de 70% de fibre de sucre de canne et de 30% de fibre de bambou. De telles proportions permettent d'augmenter considérablement la solidité dudit plateau.

En référence à la figure 1, les compartiments de réception 12 peuvent être juxtaposés les uns aux autres.

Des compartiments de réception 12 sont en particulier destinés à recevoir des instruments et sont séparés par une paroi 13.

Chaque paroi 13 saille d'une face principale formant le fond des compartiments 12, ici la face supérieure 10.

Chaque paroi 13 présente une denture 14, alternant dent et creux sur toute sa longueur.

Chaque paroi 13 est creuse et un logement 15 est formé dans chacune des parois 13, débouchant sur l'autre face principale, ici la face inférieure 11.

Dans l'exemple illustré, le plateau 1 comporte trois compartiments de réception 12 identiques juxtaposés les uns aux autres. Les compartiments de réception 12 sont séparés par une paroi 13, creuse, présentant une denture 14. Ainsi, le plateau 1 présente entre trois compartiments de réception 12 juxtaposés deux parois 13. La denture 14 de la première paroi 13 est en miroir de la denture 14 de la seconde paroi 13. Un premier creux de la denture 14 de la première paroi 13 est en miroir d'un premier creux de la seconde paroi 13 formant alors des points d'appui configuré pour recevoir un instrument médical.

Une paroi 13 avec une denture 14 est située entre chaque compartiment juxtaposé à un autre compartiment.

Le plateau 1 présente des motifs 15 formés sur l'une des faces principales, ici la face supérieure 10, qui forme le fond d'une partie des compartiments de réception 12. Les motifs 15 sont configurés pour créer des proéminences sur le fond desdits compartiments de réception 12. De tels motifs 15 permettent au plateau 1 de présenter une fonction antidérapante, empêchant le glissement du matériel médical logé dans lesdits compartiments de réception 12.

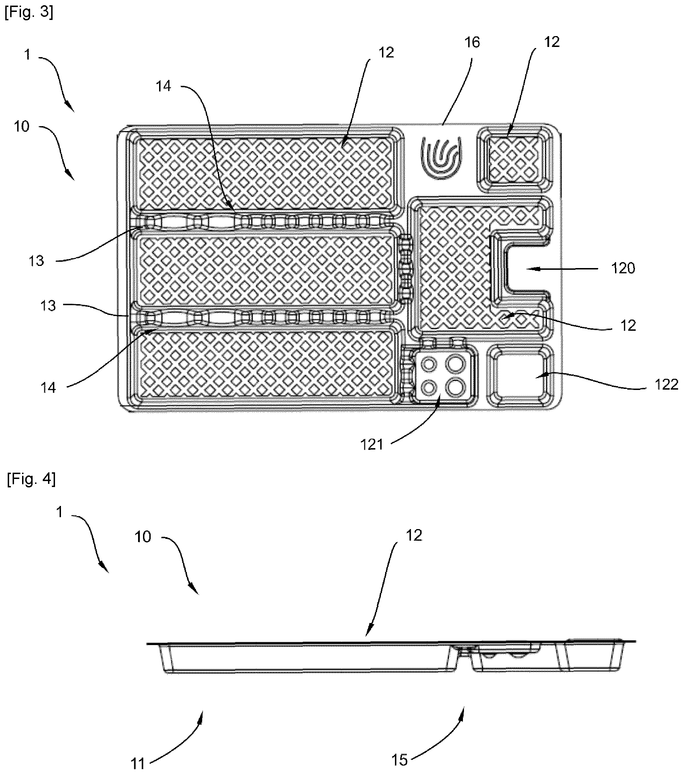

En référence aux figures 1 et 4, les compartiments de réception 12 peuvent présenter des formes et des profondeurs différentes les unes des autres, de sorte à s'adapter à une pluralité d'instruments médicaux et donc de s'adapter au mieux aux besoins de l'utilisateur.

En référence aux figures 1 et 3, le plateau 1 peut comporter une portion de préhension 16 plane. Cette portion de préhension 16 est configurée pour permettre la saisie aisée du plateau 1 par un utilisateur, ladite portion de préhension 16 étant formée par une portion plane des deux faces principales 10, 11.

Tel qu'illustré, la portion de préhension 16 peut comporter un indicateur visuel permettant d'indiquer à l'utilisateur la fonction de ladite portion de préhension 16 et l'inciter à saisir le plateau 1 par ladite portion de préhension 16.

Dans le mode de réalisation particulier de l'invention représenté sur la figure 3, le plateau 1 présente trois compartiments de réception 12, de dimensions identiques et juxtaposés les uns aux autres. Ces trois compartiments de réception 12 s'étendent sur les deux tiers de la longueur du plateau 1. Ces trois compartiments de réception 12 présentent dans leur fond un motif 15 et sont séparés les uns des autres par une paroi 13. Chacune des parois 13 présente une denture 14 alternant dent et creux sur toute la longueur de la paroi 13. Tel qu'illustré, les dimensions et l'espacement des creux de la denture 14 de chaque paroi 13 peuvent varier.

En effet, les parois 13 présentent ici deux creux juxtaposés plus larges que les autres creux des parois 13. Ces deux creux plus larges sont notamment dimensionnés pour recevoir du matériel médical spécifique tel qu'un davier, une turbine, un contre angle, etc. La denture 14 de la première paroi 13 étant en miroir de la denture 14 de la seconde paroi 13.

De plus, dans ce mode de réalisation du plateau 1, le tiers restant de la longueur du plateau 1 comporte une pluralité de compartiments de réception 120, 121, 122, une portion de préhension 16 et une portion de mélange. La pluralité de compartiments de réception 120, 121, 122 a des dimensions inférieures aux compartiments de réception 12 qui s'étendent sur les deux tiers de la longueur du plateau 1. En particulier, la pluralité de compartiments de réception 120, 121, 122 comporte quatre compartiments de réception. Deux des quatre compartiments de réception 121, 122 sont juxtaposés sur la longueur du plateau 1, et s'étendent sur le premier quart de la largeur du plateau 1, sur un bord de ce dernier.

L'un 122 des deux compartiments de réception peut par exemple présenter un fond lisse et imperméable et être configuré pour recevoir un liquide tel que de l'alcool ou de la chlorhexidine par exemple.

Le deuxième 121 peut par exemple comporter une pluralité d'alcôves de diamètres identiques ou différents configurées pour recevoir un liquide tel qu'un solvant, un vernis ou de la colle par exemple. Selon un mode de réalisation, ledit compartiment de réception 121 comporte deux alcôves de 5 mm de diamètre et deux alcôves de 9 mm de diamètre assurant la fonction de réceptacles à solvant, vernis ou colle.

Un troisième compartiment de réception 12 s'étend dans un deuxième quart de la largeur du plateau 1, sur le bord opposé des deux premiers compartiments de réception 12. Ce troisième compartiment de réception 12 est juxtaposé sur la longueur du plateau 1 à la portion de préhension 16 du plateau 1. Ce troisième compartiment de réception 12 présente un fond avec un motif 15. Ce troisième compartiment 12 peut par exemple recevoir de la petite instrumentalisation telle que des fraises.

Le quatrième compartiment de réception 12 est interposé entre les trois premiers compartiments 12 et s'étend sur un tiers de la longueur du plateau 1. Ce quatrième compartiment de réception 12 comporte un fond avec un motif 15 et présente une forme de U. Ce compartiment de réception permet par exemple de déposer compresses, cotons salivaires ou encore fil dentaire.

Entre les deux branches latérales du quatrième compartiment de réception 12 est disposé un cinquième compartiment de réception, composé d'une portion lisse 120, formant un bloc à spatuler 120. Le bloc à spatuler 120 permet au praticien de préparer un pansement dentaire tel que l'eugénate. Le bloc à spatuler 120 permet également au praticien de manipuler de la pâte à spatuler.

Dans ce mode de réalisation chaque paroi 13 interposée entre deux compartiments de réception 12, quel qu'il soit, peut présenter une denture, aux dimensions adaptées et adaptables au besoin de l'utilisateur.

L'invention concerne également un ensemble de plateaux, comportant au moins deux plateaux 1 identiques tels que précédemment décrits. Les plateaux 1 de l'ensemble de plateaux sont empilables les uns sur les autres, chaque paroi 13 d'un premier plateau 1 étant configurée pour être logée dans un logement 15 d'une paroi 13 d'un deuxième plateau 1 et chaque compartiment de réception 12, 120, 121, 122 dudit deuxième plateau 1 étant configuré pour être logé dans chaque compartiment de réception 12, 120, 121, 122 dudit premier plateau 1.

Les plateaux 1 étant empilables les uns sur les autres, ils présentent un encombrement moindre pour leur transport et leur stockage.

L'invention concerne également un procédé de fabrication d'un plateau 1. Ledit procédé comportant une première étape consistant à modeler un mélange de 70% de pulpe de sucre de canne et de 30% de pulpe de bambou sur un moule métallique. Dans cette étape la pulpe de sucre de canne et la pulpe de bambou sont humides, de sorte à prendre aisément la forme du moule métallique. Le procédé comporte une deuxième étape consistant à déshydrater ledit mélange par compression et exposition à une source de chaleur.

On notera par ailleurs que l'invention n'est pas limitée aux modes de réalisations décrits précédemment. Il apparaîtra en effet à l'homme du métier que diverses modifications peuvent être apportées aux modes de réalisations décrits ci-dessus, à la lumière de l'enseignement qui vient de lui être divulgué. La déshydratation du mélange permet de retirer 95% de son humidité et d'augmenter les liaisons entre les fibres de sucre de canne et de bambou.

Une fois déshydraté, le mélange est séché au moyen de vapeur, de sorte à obtenir un ensemble modelé à la forme requise. Le séchage à la vapeur permet une économie d'énergie certaine sur le procédé de fabrication du plateau 1.

Une fois séché, l'ensemble est exposé aux rayons ultra-violets afin d'être stérilisé.

Enfin, l'ensemble est découpé aux dimensions requises de sorte à former ledit plateau 1.

Dans la présentation détaillée de l'invention qui est faite précédemment, les termes utilisés ne doivent pas être interprétés comme limitant l'invention au mode de réalisation exposé dans la présente description, mais doivent être interprétés pour y inclure tous les équivalents dont la prévision est à la portée de l'homme du métier en appliquant ses connaissances générales à la mise en oeuvre de l'enseignement qui vient de lui être divulgué.

## Revendications

1. Plateau (1) d'examen jetable pour le domaine médical, en particulier pour le domaine dentaire, comprenant deux faces principales (10, 11) opposées, à savoir une face supérieure (10) et une face inférieure (11), ledit plateau étant monobloc et comportant un relief, formé dans l'une des faces principales (10 ; 11) et délimitant des compartiments de réception (12, 120, 121, 122), lesdits compartiments de réception (12, 120, 121, 122) étant configurés pour recevoir des instruments médicaux ; ledit plateau (1) étant formé de fibre de sucre de canne et de fibre de bambou et étant configuré pour être compostable.

2. Plateau (1) selon la revendication 1, étant formé de 70% de fibre de sucre de canne et de 30% de fibre de bambou.

3. Plateau (1) selon l'une des revendications 1 à 2, dans lequel des compartiments de réception (12) juxtaposés sont séparés par une paroi (13) présentant une denture (14) alternant dent et creux sur toute la longueur de la paroi (13).

4. Plateau (1) selon la revendication 3, dans lequel chaque paroi (13) saille de la face principale (10 ;11) formant le fond des compartiments de réception (12), ladite paroi (13) étant creuse, un logement (15) étant formé dans chaque paroi (13) débouchant sur l'autre face principale (11 ; 10).

5. Plateau (1) selon l'une des revendication 3 à 4, dans lequel au moins trois compartiments de réception (12) sont juxtaposés et séparés par deux parois (13) présentant chacune une denture (14), la denture (14) de la première paroi (13) étant en miroir de la denture (14) de la seconde paroi (13), un premier creux de la première paroi (13) en miroir d'un premier creux de la seconde paroi (13) formant alors des points d'appui configuré pour recevoir un instrument médical.

6. Plateau (1) selon l'une des revendications 1 à 5, dans lequel des motifs (15) sont formés dans la face principale (10 ;11) formant le fond d'au moins une partie des compartiments de réception (12), les motifs (15) étant configurés pour créer des proéminences sur le fond des compartiments de réception (12).

7. Plateau (1) selon l'une de revendications 1 à 6, dans lequel les compartiments de réception (12, 120, 121, 122) présentent des profondeurs différentes et des formes différentes et/ou comprennent un compartiment de réception (122) ayant un fond lisse et/ou un compartiment de réception (121) comprenant une pluralité d'alcôves, notamment deux paires d'alcôves de diamètre différent, et/ou un compartiment de réception formant un bloc à spatuler (120).

8. Plateau (1) selon l'une des revendications 1 à 7, comportant une portion de préhension (16) plane, configurée pour permettre la saisie dudit plateau (1) par un utilisateur, ladite portion de préhension (16) étant formée par une portion plane des deux faces principales (10, 11).

9. Procédé de fabrication d'un plateau (1) selon l'une des revendications 1 à 8, comportant les étapes suivantes :
- modeler un mélange de 70% de pulpe de sucre de canne et de 30% de pulpe de bambou sur un moule métallique ;
- déshydrater ledit mélange par compression et exposition à une source de chaleur ;
- sécher ledit mélange au moyen de vapeur de sorte à obtenir un ensemble modelé à la forme requise ;
- exposer ledit ensemble aux rayons ultra-violets ; et
- découper ledit ensemble traité aux rayons ultra-violets aux dimensions requises de sorte à former ledit plateau (1).

10. Ensemble de plateaux, comportant au moins deux plateaux (1) identiques selon l'une des revendications 1 à 8, dans lequel lesdits plateaux (1) sont empilables les uns sur les autres, chaque paroi (13) d'un premier plateau (1) étant configurée pour être logée dans un logement (15) d'une paroi (13) d'un deuxième plateau (1) et chaque compartiment de réception (12, 120, 121, 122) dudit deuxième plateau (1) étant configuré pour être logé dans chaque compartiment de réception (12, 120, 121, 122) dudit premier plateau (1).
